# FASCICULE DE BREVET EUROPEEN

(11) **EP 1 237 552 B1**
(45) Date de publication et mention de la délivrance du brevet: **17.08.2005**
(21) Numéro de dépôt: 00981467.4
(22) Date de dépôt: 28.11.2000
(51) Int. Cl.: A61K 31/48, A61K 31/425, A61P 25/00, A61P 21/00

(54) **ASSOCIATION DE LUMILYSERGOL ET RILUZOLE POUR LA PREVENTION ET/OU LE TRAITEMENT DE MALADIES MOTONEURONALES**
KOMBINATION VON LUMILYSERGOL UND RILUZOL FÜR DIE VORBEUGUNG UND/ODER BEHANDLUNG VON MOTONEURONALEN ERKRANKUNGEN
COMBINATION OF LUMILYSERGOL AND RILUZOLE FOR PREVENTING AND/OR TREATING MOTONEURONAL DISEASES

(30) Priorité: 01.12.1999 FR 9915139
(43) Date de publication de la demande: 11.09.2002
(60) Demande divisionnaire: 04014547.6
(73) Titulaire: Aventis Pharma S.A., 92160 Antony (FR)
(72) Inventeur: DIB, Michel, F-75013 Paris (FR); STUTZMANN, Jean-Marie, F-94440 Villecresnes (FR)
(74) Mandataire: Rousseau, Pierrick Edouard
(86) Numéro de dépôt international: PCT/FR2000/003314
(87) Numéro de publication internationale: WO 2001/039776

(56) Documents cités:
- WO-A-00/30642
- WO-A-99/34785
- DE-A- 4 240 798
- US-A- 5 780 489
- SANDERINK G J ET AL: "Involvement of human CYP1A isoenzymes in the metabolism and drug interactions of riluzole in vitro" JOURNAL OF PHARMACOLOGY AND EXPERIMENTAL THERAPEUTICS,US,AMERICAN SOCIETY FOR PHARMACOLOGY AND, vol. 282, no. 3, septembre 1997 (1997-09), pages 1465-1472, XP002112642 ISSN: 0022-3565
- MARTINET M ET AL: "Pharmacokinetics and metabolism of riluzole" DRUGS OF TODAY / MEDICAMENTOS DE ACTUALIDAD,ES,J.R. PROUS SS.A. INTERNATIONAL PUBLISHERS, vol. 33, no. 8, octobre 1997 (1997-10), pages 587-594, XP002112643 ISSN: 0025-7656
- LOUVEL E ET AL: "Therapeutic advances in amyotrophic lateral sclerosis" TRENDS IN PHARMACOLOGICAL SCIENCES,GB,ELSEVIER TRENDS JOURNAL, CAMBRIDGE, vol. 18, no. 6, 1 juin 1997 (1997-06-01), pages 196-203, XP004094506 ISSN: 0165-6147

## Description

La présente invention concerne l'utilisation de l'association de lumilysergol et de riluzole ou un de ses sels pharmaceutiquement acceptables dans la prévention et/ou le traitement des maladies motoneuronales.

Le riluzole est commercialisé pour le traitement de la sclérose latérale amyotrophique. Ce composé est également utile comme anticonvulsivant, anxiolytique et hypnotique (EP50551), dans le traitement de la schizophrénie (EP305276), dans le traitement des troubles du sommeil et de la dépression (EP305277), dans le traitement des désordres cérébrovasculaires et comme anesthésique (EP282971), dans le traitement des traumatismes spinaux, crâniens ou crânio-spinaux (WO94/13288), comme radiorestaurateur (WO94/15600), dans le traitement de la maladie de Parkinson (WO94/15601), dans le traitement du neuro-sida (WO94/20103), dans le traitement des maladies mitochondriales (WO95/19170).

Une association de riluzole avec un précurseur ou dérivé de la glutathione est connue pour son utilisation dans le traitement de la sclérose latérale amyotrophique (US 5 780 489).

Le lumilysergol ou 1,6-diméthyl-8β-hydroxyméthyl-10α-méthoxyergoline ou 1-méthyl-10α-méthoxy-9,10-dihydrolysergol est un des métabolites de la nicergoline (F. ARCAMONE et coll., Biochem. Pharmacol., 21 (16), 2205-2013 (1972)). Ce composé présente comme la nicergoline mais à un degré moindre des propriétés α1-adrénergiques et 5HT1a sérotoninergiques. Il est également utile comme intermédiaire pour la préparation de la nicergoline (brevet FR2616788).

L'association de riluzole et de nicergoline a déjà été décrite comme utile dans le traitement de la spasticité (WO 00/30642).

Il est connu que, in vitro, les motoneurones cultivés sans facteur trophique meurent dans les 48 à 72 heures (AG. ESTEVEZ et coll.,J. Neurosci., 18 (3). 923-931 (1998) et 18 (10), 3708-3714 (1998)).

Par ailleurs, la mort motoneuronale induite par privation de facteur trophique peut être partiellement prévenue quand les motoneurones sont cultivés sur des monocouches d'astrocytes ou en présence de milieu conditionné obtenu à partir d'astrocytes. En outre, la production par les astrocytes d'activité trophique pour les motoneurones est stimulée par le riluzole (H. PELUFFO et coll., Neuroscience letters, 228, 207-211 (1997)).

Il a maintenant été trouvé que l'association de riluzole ou un de ses sels pharmaceutiquement acceptables et de lumilysergol agit de manière synergique et augmente fortement l'activité trophique secrétée par les astrocytes. Cette association peut ainsi être utilisée dans la prévention et/ou le traitement des maladies motoneuronales.

Les maladies motoneuronales incluent notamment la sclérose latérale amyotrophique, l'atrophie musculaire spinale progressive, l'atrophie musculaire infantile, la slérose latérale primaire.

Le protocole général utilisé est décrit par H. PELUFFO et coll., Neuroscience letters, 228, 207-211 (1997).

### CULTURES ENRICHIES EN MOTONEURONES :

Les cultures enrichies en motoneurones sont préparées en utilisant la méthode de centrifugation décrite par R.L. SCHNAAR et A.E. SCHAFFNER, J. Neurosci., 1, 204-217 (1981) et modifiée par W. CAMU et C.E. HENDERSON, J. Neurosci. Methods, 44, 59-70 (1992). Sur des plaques de cultures préalablement enduites de laminine/ornithine selon la méthode de A.G. ESTEVEZ et coll., J. Neurosci., 18 (3), 923-931 (1998), on étale les motoneurones à une densité de 2500 cellules par plaque de 35 mm. Les cultures sont ensuite maintenues dans du milieu L15 (GIBCO BRL) contenant du bicarbonate de sodium (22mM), de la conalbumine (0,1 mg/ml), de la putrescine (0,1 mM), de l'insuline (5 µg/ml), du sélénite de sodium (31 nM), du glucose (20 mM), de la progestérone (21 nM), de la pénicilline (100 IU/ml) et de la streptomycine (100 ug/ml).

Les motoneurones ainsi obtenus sont composés de neurones larges (25-30 µM) et homogènes avec de longs neurites branchés. Plus de 70% des cellules sont immunoréactives pour le récepteur neurotrophine p75 et les marqueurs Islet ½ pour les motoneurones spinaux. Environ 70% des motoneurones meurent par apoptose 24 heures après l'étalement si la culture est effectuée en absence de facteur trophique.

### CULTURES D'ASTROCYTES DE MOELLE EPINIERE :

Les astrocytes sont obtenus à partir de jeunes rats Wistar âgés d'un jour selon la méthode de R.P. SANETO et J. DE VELLIS, in Neurochemistry a practical approach (A.J. TURNER et H.S. St JOHN) IRL Press, Oxford-Washington DC, p27-63 (1987) légérement modifiée (H. PELUFFO et coll., Neuroscience Letters, 228, 207-211 (1997). Les moëlles épinières sont disséquées stérilement, débarassées des méninges et des ganglions dorsaux et coupées avant incubation à 37°C pendant 25 minutes dans du PBS (phosphate buffer saline : NaCl 137 mM, KCl 2,68 mM, Na₂HPO₄ 6,45 mM, KH₂PO₄ 1,47 mM) auquel on a ajouté 0,25% de trypsine. Le traitement enzymatique est stoppé par addition de 10 ml de milieu Dubelcco-Eagle modifié (DMEM) auquel on a ajouté 10% de sérum foetal de veau (FCS) et la dissociation mécanique est complétée en utilisant l'extrémité d'une pipette de 1 ml. Les cellules sont collectées par centrifugation puis étalées à une densité de 1,5-2x10⁶ cellules pour 25 cm² de milieu de culture dans du DMEM auquel on a ajouté 10% de FCS, 100UI/ml de pénicilline et 100ug/ml de streptomycine. Après 3 jours in vitro les cultures sont alimentées chaque jour. Quand une monocouche visible de cellules est terminée, les cultures sont agitées 48 heures à 250 rpm et les monocouches sont traitées par du cytosine arabinoside (10⁻⁵ M) pendant 48 heures. Les monocouches d'astrocytes ainsi obtenues sont maintenues 48 heures en milieu de culture puis amplifiées à une densité de 2x10⁴ cellules/cm².

Les monocouches d'astrocytes montrent une pureté de plus de 98% déterminée par l'immunoréactivité pour la protéine gliale fibrilaire acide (GFAP).

### TRAITEMENT DES ASTROCYTES AVEC LES PRODUITS A TESTER

Le traitement des astrocytes avec les produits à tester s'effectue de la manière suivante : l'ergoline est dissoute dans de l'éthanol absolu et le riluzole dans HCl 0,01N, stérilisé par filtration et utilisé immédiatement après préparation. Les monocouches d'astrocytes sont exposées au véhicule ou aux solutions des produits à tester pendant 24 heures. Ce milieu est utilisé à une dilution de 10 fois dans du milieu L15 (Dubelco) frais. Les monocouches d'astrocytes sont lavées 3 fois avec du DMEM et incubées avec du milieu complet L15. Le milieu conditionné d'astrocytes est repris 24 heures plus tard et centrifugé à 2000 g pendant 3 minutes et utilisé immédiatement ou conservés à -70°C 2 semaines maximum sans perte d'activité trophique.

Pour le marquage immunochimique des motoneurones, les cultures sont fixées dans du méthanol glacé pendant 15 minutes puis lavées et les sites non spécifiques sont bloqués avec 2% d'albumine de sérum bovin (BSA) + 0,1 % de triton X100 dans du PBS. Les cultures sont incubées successivement avec des anticorps dirigées contre les sous-unités 200kD de neurofilaments (1:200 Amersham), du sérum de chèvre biotyliné (1:125, Gibco) et du peroxydase de streptavidine (1:200, Gibco) pendant 60 minutes à température ambiante.

### COMPTAGE DES CELLULES ET ANALYSE STATISTIQUE

Les cellules immunoréactives pour les neurofilaments et exhibant des neurites plus longs que les diamètres des cellules sont considérés comme des motoneurones viables. Le nombre de motoneurones est évalué par comptage des cellules marquées dans une surface de 0,4-1 cm² sous microscope grossissant 200 fois. Dans tous les cas, les valeurs sont exprimées comme un pourcentage du nombre de motoneurones présents dans les cultures maintenues avec des facteurs trophiques (BDNF). Les expériences sont réalisées au moins 3 fois.

Les analyses statistiques sont effectuées en utilisant le test de Student (t-test).

Les résultats obtenus sont les suivants :

Effet du riluzole, et de lumilysergol seuls et leurs associations sur l'activité neurotrophique des motoneurones produite par les astrocytes spinaux :

| PRODUITS | Survie motoneuronale en % |
|---|---|
| véhicule seul | 44±1,41 |
| riluzole (0,1 µM) | 68,0±3 |
| lumilysergol (0,1 µM) | 57,4±3,53 |
| riluzole 0,1 µM +lumilysergol 0,1 µM | 84,5±2 |

Ces résultats démontrent que l'association riluzole et lumilysergol stimule de manière synergistique l'activité trophique motoneuronale produite par les monocouches d'astrocytes spinaux.

Le riluzole peut être préparé selon le procédé décrit dans le brevet EP50551.

Comme sels pharmaceutiquement acceptables du riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Le 1,6-diméthyl-8β-hydroxyméthyl-10α-méthoxyergoline peut être préparé selon la méthode décrite dans le brevet FR2616788.

La présente invention concerne également les compositions pharmaceutiques comprenant l'association de riluzole ou un de ses sels pharmaceutiquement acceptables et de lumilysergol à l'état pur ou sous forme d'une association avec un ou plusieurs diluants et/ou adjuvants compatibles et pharmaceutiquement acceptables et/ou éventuellement en association avec un autre produit pharmaceutiquement compatible et physiologiquement actif. Les produits qui constituent l'association peuvent être administrés simultanément, séparément ou d'une manière étalée dans le temps de façon à obtenir le maximum d'efficacité de l'association.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.
Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.
Les compositions pour administration rectale sont les suppositoires ou les capsules rectales qui contiennent, outre le produit actif, des excipients tels que le beurre de cacao, des glycérides semisynthétiques ou des polyéthylèneglycols.

La présente invention concerne également l'utilisation de l'association de riluzole ou un de ses sels pharmaceutiquement acceptables et de lumilysergol pour la préparation d'un médicament notamment utile dans la prévention et/ou le traitement des maladies motoneuronales et notamment la sclérose latérale amyotrophique, l'atrophie musculaire spinale progressive, l'atrophie musculaire infantile, la sclérose latérale primaire.
L'invention concerne également la méthode de prévention et/ou de traitement des maladies motoneuronales et notamment la sclérose latérale amyotrophique, l'atrophie musculaire spinale progressive, l'atrophie musculaire infantile, la slérose latérale primaire qui consiste à administrer au patient une association de riluzole ou un de ses sels pharmaceutiquement acceptables et de lumilysergol, soit simultanément soit séparément soit de manière étalée dans le temps.

Les doses dépendent de l'effet recherché, de la durée du traitement et de la voie d'administration utilisée; elles sont généralement de 50 mg à 200 mg par jour de riluzole et 30 mg à 120 mg par jour de lumilysergol.

La présente invention concerne également les associations dans laquelle on utilise 50 à 200 parties en poids de riluzole pour 30 à 120 parties en poids de lumilysergol.

## Revendications

1. Utilisation de l'association de riluzole ou un de ses sels pharmaceutiquement acceptables et du lumilysergol pour la préparation d'un médicament utile pour la prévention et/ou le traitement des maladies motoneuronales.

2. Utilisation selon la revendication 1 pour la préparation d'un médicament contenant 50 à 200 parties en poids de riluzole pour 30 à 120 parties en poids de lumilysergol.

3. Utilisation selon l'une des revendications 1 ou 2 pour la préparation d'un médicament pour une utilisation simultanée, séparée ou étalée dans le temps.

4. Utilisation selon l'une des revendication 1 à 3 pour la préparation d'un médicament pour la prévention et/ou le traitement de la sclérose latérale amyotrophique, l'atrophie musculaire spinale progressive, l'atrophie musculaire infantile, la slérose latérale primaire.

5. Association de riluzole ou un de ses sels pharmaceutiquement acceptables et de lumilysergol.

6. Association selon la revendication 5 dans laquelle on utilise 50 à 200 parties en poids de riluzole pour 30 à 120 parties en poids de lumilysergol.

7. Composition pharmaceutique constituée d'une association selon la revendication 5 à l'état pur ou en présence de tout diluant ou adjuvant compatible et pharmaceutiquement acceptable.

## Patentansprüche

1. Verwendung einer Kombination von Riluzol oder eines seiner pharmazeutisch akzeptablen Salze und Lumilysergol für die Herstellung eines Arzneimittels, das zur Vorbeugung und /oder Behandlung von motoneuronalen Erkrankungen verwendbar ist.

2. Verwendung nach Anspruch 1 für die Herstellung eines Arzneimittels, enthaltend 50 bis 200 Gewichtsteile Riluzol für 30 bis 120 Gewichtsteile Lumilysergol.

3. Verwendung nach den Ansprüchen 1 oder 2 für die Herstellung eines Arzneimittels zur zeitlich gleichzeitigen, unabhängigen oder aufeinander folgenden Verwendung.

4. Verwendung nach den Ansprüchen 1 bis 3 für die Herstellung eines Arzneimittels zur Vorbeugung und/oder Behandlung der amyotrophischen Lateralsklerose, spinalen progressiven Muskelatrophie, infantilen Muskelatrophie, primären Lateralsklerose.

5. Kombination von Riluzol oder eines seiner pharmazeutisch akzeptablen Salze und Lumilysergol.

6. Kombination nach Anspruch 5, worin 50 bis 200 Gewichtsteile Riluzol für 30 bis 120 Gewichtsteile Lumilysergol verwendet werden.

7. Pharmazeutische Zusammensetzung bestehend aus einer Kombination nach Anspruch 5 in reiner Form oder in Anwesenheit jedes Verdünnungs- oder Zusatzmittels, das kompatibel und pharmazeutisch akzeptabel ist.

## Claims

1. Use of the combination of riluzole or one of its pharmaceutically acceptable salts and of lumilysergol, for the preparation of a medicament useful for the prevention and/or treatment of motoneuron diseases.

2. Use according to Claim 1, for the preparation of a medicament containing 50 to 200 parts by weight of riluzole per 30 to 120 parts by weight of lumilysergol.

3. Use according to either of Claims 1 and 2, for the preparation of a medicament for use simultaneously, separately or spaced out over time.

4. Use according to one of Claims 1 to 3, for the preparation of a medicament for the prevention and/or treatment of amyotrophic lateral sclerosis, progressive spinomuscular atrophy, infantile muscular atrophy, primary lateral sclerosis.

5. Combination of riluzole or one of its pharmaceutically acceptable salts and of lumilysergol.

6. Combination according to Claim 5, in which 50 to 200 parts by weight of riluzole are used per 30 to 120 parts by weight of lumilysergol.

7. Pharmaceutical composition consisting of a combination according to Claim 5, in the pure state or in the presence of any compatible and pharmaceutically acceptable diluent or adjuvant.
